# EUROPÄISCHE PATENTSCHRIFT

(11) **EP 4 252 705 B1**
(45) Veröffentlichungstag und Bekanntmachung des Hinweises auf die Patenterteilung: **23.07.2025**
(21) Anmeldenummer: 22165823.0
(22) Anmeldetag: 31.03.2022
(51) Int. Cl.: A61C 13/00, B33Y 10/00, B33Y 30/00, B33Y 50/00, B33Y 50/02, B33Y 80/00

(54) **HERSTELLUNGSVERFAHREN FÜR EIN DENTALOBJEKT**
METHOD FOR MANUFACTURING A DENTAL OBJECT
PROCÉDÉ DE FABRICATION D'UN OBJET DENTAIRE

(43) Veröffentlichungstag der Anmeldung: 04.10.2023
(73) Patentinhaber: Ivoclar Vivadent AG, 9494 Schaan (LI)
(72) Erfinder: FREY, Alen, 6800 Feldkirch (AT); JOHN, Hendrik, 9470 Buchs (CH); SCHÜLKE, Andreas, 9472 Grabs (CH); HÖNOW, Katja, 9487 Gamprin (LI)
(74) Vertreter: Baldus, Oliver

(56) Entgegenhaltungen:
- US-A1- 2006 008 774
- US-B2- 10 856 957

## Beschreibung

Die vorliegende Erfindung betrifft ein Herstellungsverfahren für ein Dentalobjekt und ein Herstellungssystem zum Herstellen eines Dentalobjekts.

In bekannten digitalen Arbeitsabläufen werden in der CAD-Software dentale Restaurationen erzeugt und in einem 3D-CAD-Dateiformat abgespeichert. In Abhängigkeit von der verwendeten CAD-Software und dem sich anschließenden Arbeitsablauf können zusammen mit der 3D-CAD-Datei sogenannte Metadaten erzeugt und abgelegt werden, die zusätzliche Informationen umfassen. Allerdings kann für die Herstellung eines Dentalobjektes eine Anpassung einer Orientierung erforderlich sein.

Metadaten können hilfreich sein, um die abtragenden Fertigungsprozesse zu steuern oder optimieren, wie beispielsweise Frässtrategien für eine speziellen Restaurationstyp zuordnen zu können. In dreidimensionalen Druckverfahren, bei denen Stützstrukturen erforderlich sind, wie beispielsweise in der Stereolithografie, einer VAT-Polymerisation, einem 3D-DLP-Druck, oder einem selektiven Laser-Sintern von Metallen, sollten bestimmte Flächen von dentalen Restaurationen frei von der Anbindung von Stützstrukturen sein, um eine Präzision und Flächenqualität positiv zu beeinflussen.

Bei Metadaten besteht jedoch der Nachteil, dass diese bereits zuvor in der CAD-Software erzeugt werden und es sich um proprietäre Datenformate handelt. In diesem Fall muss eine verwendete CAM-Software die zugeordneten proprietären Metadaten importieren und interpretieren können. Da diese Metadaten-Formate nicht universell sind, muss für jedes proprietäre Metadaten-Format eine eigene Schnittstelle programmiert werden. Liegen keine Metadaten zu einem Objekt vor, kann die CAM-Software beim Import nicht bestimmen, um welche Indikation es sich handelt.

Die Druckschrift US 10,856,957 B2 betrifft ein Verfahren zum Herstellen eines dreidimensionalen digitalen Modells einer Prothesenbasis zur Herstellung unter Verwendung einer auf Licht basierenden dreidimensionalen Druckvorrichtung, umfassend das Positionieren eines virtuellen Referenzmodells neben einer von einer Software bereitgestellten virtuellen Bauplattformoberfläche.

Die Druckschrift US 2006/008774 A1 betrifft ein Verfahren zur Herstellung eines Zahnersatzteils aus einem Rohling unter Erstellung eines Bearbeitungsplans für Bearbeitungsgeräte sowie eine Vorrichtung zur Herstellung eines Zahnersatzteils.

Es ist die technische Aufgabe der Erfindung, ein digitales Dentalobjekt so zu orientieren, dass dieses anschließend effizient in einem Herstellungsverfahren hergestellt werden kann. Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Herstellungsverfahren für ein Dentalobjekt gelöst, mit den Schritten eines Erzeugens eines digitalen Dentalobjekts in einem Koordinatensystem, das eine Form des herzustellenden Dentalobjekts beschreibt; eines Drehens der räumlichen Orientierung des digitalen Dentalobjektes in dem Koordinatensystem auf Basis eines digitalen Referenzobjektes in dem Koordinatensystem, wobei die Orientierung des digitalen Dentalobjektes in dem Koordinatensystem gedreht wird, bis eine räumliche Abweichung zwischen dem digitalen Dentalobjekt und dem digitalen Referenzobjekt einen vorgegebenen Wert unterschreitet.; und eines Herstellens des Dentalobjekts auf Basis des digitalen Dentalobjekts mit der gedrehten räumlichen Orientierung. Durch das Herstellungsverfahren wird eine automatische optimierte, fertigungsspezifische Orientierung dentaler Indikationen für den digitalen Fertigungsprozess erreicht. Der Wert der Abweichung kann aus einem räumlichen Überlappungsbereich der beiden Objekte bestimmt werden. Dadurch wird beispielsweise der technische Vorteil erreicht, dass das digitale Dentalobjekt und das digitale Referenzobjekt in einem iterativen Verfahren aneinander angenähert werden können.

In einer technisch vorteilhaften Ausführungsform des Herstellungsverfahrens wird eine Größe des digitalen Referenzobjekts an eine Größe des digitalen Dentalobjektes und/oder eine Größe des digitalen Dentalobjektes an eine Größe des digitalen Referenzobjekts angepasst. Die Größen des digitalen Referenzobjekts und des digitalen Dentalobjektes werden so durch gleichförmige Skalierung aneinander angepasst, dass sie sich möglichst ähnlich sind. Dadurch wird beispielsweise der technische Vorteil erreicht, dass eine höhere Übereinstimmung oder Überlappung zwischen dem digitalen Dentalobjekt und dem digitalen Referenzobjekt erzielt wird.

In einer weiteren technisch vorteilhaften Ausführungsform des Herstellungsverfahrens wird eine Position des digitalen Dentalobjektes in dem Koordinatensystem auf Basis eines digitalen Referenzobjektes in dem Koordinatensystem verschoben. Dadurch wird beispielsweise ebenfalls der technische Vorteil erreicht, dass eine höhere Übereinstimmung oder Überlappung zwischen dem digitalen Dentalobjekt und dem digitalen Referenzobjekt erzielt wird.

In einer weiteren technisch vorteilhaften Ausführungsform des Herstellungsverfahrens wird die Position des digitalen Dentalobjektes in dem Koordinatensystem verschoben, bis eine räumliche Abweichung zwischen dem digitalen Dentalobjekt und dem digitalen Referenzobjekt einen vorgegebenen Wert unterschreitet. Dadurch wird beispielsweise ebenfalls der technische Vorteil erreicht, dass das digitale Dentalobjekt und das digitale Referenzobjekt in einem iterativen Verfahren aneinander angenähert werden können.

In einer weiteren technisch vorteilhaften Ausführungsform des Herstellungsverfahrens wird das Dentalobjekt mittels eines additiven Herstellungsverfahrens hergestellt. Dadurch wird beispielsweise der technische Vorteil erreicht, dass das Dentalobjekt auf einfache Weise in einer beliebigen Form hergestellt werden kann.

In einer weiteren technisch vorteilhaften Ausführungsform des Herstellungsverfahrens wird zu dem digitalen Dentalobjekt mit der gedrehten räumlichen Orientierung eine digitale Stützstruktur auf Basis der Orientierung des Referenzobjektes hinzugefügt. Das Hinzufügen der Stützstruktur kann unabhängig vom Referenzobjekt erfolgen. Dadurch wird beispielsweise der technische Vorteil erreicht, dass die digitale Stützstruktur automatisch an einer vorgesehenen und geeigneten Stelle hinzugefügt wird. Dadurch kann die Herstellung des Dentalobjekt verbessert und beschleunigt werden. Dadurch wird beispielsweise zusätzlich der technische Vorteil erreicht, dass die Stützstruktur automatisch an vorgesehenen und geeigneten Flächen angeordnet werden können.

In einer weiteren technisch vorteilhaften Ausführungsform des Herstellungsverfahrens wird das Dentalobjekt mittels eines subtraktiven Herstellungsverfahrens hergestellt. Dadurch wird beispielsweise ebenfalls der technische Vorteil erreicht, dass das Dentalobjekt auf einfache Weise hergestellt werden kann.

In einer weiteren technisch vorteilhaften Ausführungsform des Herstellungsverfahrens wird zu dem digitalen Dentalobjekt mit der gedrehten räumlichen Orientierung eine digitale Haltestruktur auf Basis der Orientierung des Referenzobjektes hinzugefügt. Dadurch wird beispielsweise der technische Vorteil erreicht, dass Haltestege zum Halten eines herausgefrästen Dentalobjekts automatisch lediglich an geeigneten Stellen des Dentalobjekts angeordnet sind. Dadurch wird beispielsweise der technische Vorteil erreicht, dass die Haltestruktur automatisch an vorgesehenen und geeigneten Flächen angeordnet werden können.

In einer weiteren technisch vorteilhaften Ausführungsform des Herstellungsverfahrens ist das digitale Referenzobjekt aus einer Datenbank mit mehreren digitale Referenzobjekten abrufbar. Für unterschiedliche Herstellungsverfahren kann ein unterschiedliches Referenzobjekt abrufbar sein, wie beispielsweise einen 3D-Druck, ein Pressverfahren, ein Gussverfahren oder eine Nachvergütung. In diesem Fall wird zunächst das Herstellungsverfahren ausgewählt und das Referenzobjekt für das Herstellungsverfahren aus der Datenbank abgerufen. Dadurch wird beispielsweise der technische Vorteil erreicht, dass je nach Herstellungsverfahren eine optimale Orientierung des Dentalobjektes erreicht wird.

In einer weiteren technisch vorteilhaften Ausführungsform des Herstellungsverfahrens umfasst das digitale Dentalobjekt Daten über eine Indikation. Dadurch wird beispielsweise der technische Vorteil erreicht, dass je nach Indikation ein passendes Referenzobjekt ausgewählt werden kann.

In einer weiteren technisch vorteilhaften Ausführungsform des Herstellungsverfahrens ist das digitale Referenzobjekt aus einer Datenbank auf Basis der Daten über die Indikation abrufbar oder kann zugeordnet werden. Das digitale Referenzobjekt kann auch manuell ausgewählt oder bestimmt werden. Dadurch wird beispielsweise der technische Vorteil erreicht, dass unterschiedliche Referenzobjekte für unterschiedliche Indikationen abgerufen und/oder dem digitalen Dentalobjekt zugeordnet werden können.

Gemäß einem zweiten Aspekt wird die technische Aufgabe durch ein Herstellungssystem zum Herstellen eines Dentalobjekts gelöst, mit einem Computerprogramm, das Befehle umfasst, die bei der Ausführung des Computerprogramms durch einen Computer diesen veranlassen, das Verfahren nach dem ersten Aspekt auszuführen. Dadurch werden die gleichen technischen Vorteile, wie durch das Verfahren nach dem ersten Aspekt erreicht.

Ausführungsbeispiele der Erfindung sind in den Zeichnungen dargestellt und werden im Folgenden näher beschrieben.

Es zeigen:
- Fig. 1: eine schematische Ansicht des Herstellungsverfahrens für ein Dentalobjekt;
- Fig. 2: ein Dentalobjekt mit Stütz- oder Haltestrukturen; und
- Fig. 3: ein Blockdiagramm eines Herstellungsverfahrens für ein Dentalobjekt.

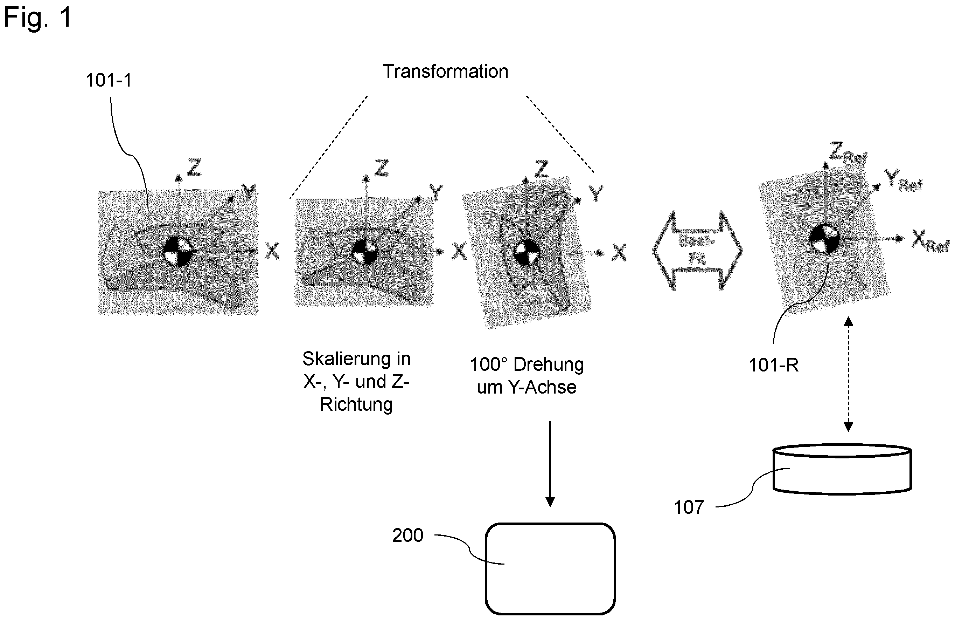

Fig. 1 zeigt eine schematische Ansicht des Herstellungsverfahrens für ein reales Dentalobjekt 100-2 auf Basis eines digitalen Dentalobjektes 100-1. Das reale Dentalobjekt 100-2 ist beispielsweise eine Krone, eine Brücke, ein Veneer, ein Abutment, ein Inlay, ein Onlay, eine Schiene oder eine Teil- oder Vollprothese. Im Allgemeinen kann das Dentalobjekt 100-2 jedes Objekt im Dentalbereich sein, das im Rahmen eines dreidimensionalen Herstellungsverfahrens hergestellt werden soll, wie beispielsweise in einem 3D-Druckverfahren oder Fräsverfahren.

Zunächst erzeugt die CAD-Software ein digitales Dentalobjekt 100-1 in einem Koordinatensystem, das eine Form des herzustellenden Dentalobjekts 100-2 beschreibt. Das digitale Dentalobjekt 100-1 ist zunächst in einer beliebigen Orientierung in dem Koordinatensystem orientiert. Das digitale Dentalobjekt 100-1 dient einer bestimmten Indikation. Die Form des digitalen Dentalobjekts 100-1 ist beispielsweise durch eine Menge von Dreiecken oder Koordinaten angegeben, die die Oberfläche des digitalen Dentalobjekt 100-1 festlegen. Das digitale Dentalobjekt 100-1 kann in einem Datensatz gespeichert sein.

Eine Datenbank 107 umfasst eine Mehrzahl von digitalen Referenzobjekten 100-R, denen beispielsweise jeweils eine Indikation und ein Herstellungsverfahren zugeordnet ist.

Das Referenzobjekt 100-R gibt eine Referenzform mit einer bestimmten Referenzposition und -orientierung innerhalb des Koordinatensystems an. Die Form des digitalen Dentalobjekts 100-R ist beispielsweise ebenfalls durch eine Menge von Dreiecken oder Koordinaten angegeben, die die Oberfläche des Referenzobjektes 100-R festlegen. Auch das digitale Referenzobjekt 100-R kann in einem Datensatz gespeichert sein.

Zu dem Referenzobjekt 100-R können neben der Indikation folgende Informationen in der Datenbank 107 abgelegt werden:
- Beschreibung der Orientierung des Referenzobjekts 100-R durch zahntechnische Begrifflichkeiten;
- Welche Flächen 109-1 mit Stütz- oder Haltestrukturen 103 oder 105 belegt werden dürfen (Support-Gebot); und
- Welche Flächen 109-2 nicht mit Stütz- oder Haltestrukturen 103 oder 105 belegt werden dürfen (Support-Verbot).

Die Flächen 109-1 und 109-2, die Gebote und Verbote für eine Stütz- oder Haltestruktur auf dem Referenzobjekt 100-R beschreiben, können auf das digitale Dentalobjekt 100-1 übertragen werden.

Aus der Datenbank 107 wird ein digitales Referenzobjekt 100-R einer Indikation geladen, die der Indikation des digitalen Dentalobjekts 100-1 entspricht. Ist die Indikation des digitalen Dentalobjekts 100-1 beispielsweise ein Inlay wird aus der Datenbank 107 beispielsweise das digitales Referenzobjekt 100-R für ein Inlay geladen.

Wenn sowohl das digitale Dentalobjekt 100-1 als auch das Referenzobjekt 100-R geladen sind, wird das digitale Dentalobjekt 100-1 auf eine ähnliche Größe wie das Referenzobjekt 100-R skaliert oder umgekehrt. Alternativ können das Dentalobjekt 100-1 und das Referenzobjekt 100-R auf eine gemeinsame ähnliche Größe skaliert werden. Dabei sollte sich das Seitenverhältnis nicht verändern. Anschließend werden unterschiedliche Dreh- oder Verschiebungsoperationen auf dem digitalen Dentalobjekt 100-1 durchgeführt, wie beispielsweise eine Drehung des digitalen Dentalobjekt 100-1 um eine Y-Achse. Bei einer Verschiebung können die Massenschwerpunkte des digitalen Dentalobjektes 101-1 und des Referenzobjektes 101-R überlagert werden. Die Skalierung als auch die Drehung und Verschiebung dienen dem Zweck eine maximale Überlappung zwischen dem digitalen Dentalobjekt 100-1 und dem Referenzobjekt 100-R erreichen.

Eine Überlappung kann durch Anpassung des Jaccard-Koeffizienten, des digitalen Dentalobjektes 101-1 und der Referenzobjektes 101-R berechnet werden. Zur Berechnung der Überlappung kann die räumliche Schnittmenge zwischen dem digitalen Dentalobjekt 100-1 und dem Referenzobjekt 100-R berechnet werden.

Die Skalierung und die Dreh- oder Verschiebungsoperationen können zu diesem Zweck iterativ wiederholt werden, bis eine räumliche Abweichung zwischen dem digitalen Dentalobjekt 100-1 und dem digitalen Referenzobjekt 100-R einen vorgegebenen Wert unterschreitet. Die Abweichung kann als Überdeckungsgrad auf Basis eines Volumens berechnet werden. Der Überdeckungsgrad kann eine verhältnisskalierte Zahl zwischen 0 und 1 sein, die als Prozentsatz angesehen werden kann.

Für die Berechnung werden das digitale Dentalobjekt 100-1 und das Referenzobjekt 100-R in eine Voxel-Darstellung transformiert. Anschließend werden alle leeren Voxel innerhalb beider Objekte gefüllt. Die Anzahl der Voxel, die von beiden Objekten eingenommen sind, wird durch die Anzahl der gesamten gefüllten Voxel geteilt. Dies entspricht einer Berechnung des Jaccard-Koeffizienten von zwei Mengen entspricht, die auch als Intersection over Union (IoU) bezeichnet wird. Der berechnete Wert zwischen 0 und 1 gibt an, wie weit die beiden dreidimensionalen Objekte übereinstimmen. Dieser Wert kann durch Translation und Rotation der Objekte maximiert werden.

Die Überlagerung und der iterative Abgleich erfolgen nach der Best-Fit-Methode. Dabei wird das digitale Dentalobjekt 100-1 so lange skaliert und rotatorisch und translatorisch transformiert, bis eine maximale Überlappung mit dem Referenzobjekt 100-R erzielt wird. Auf diese Weise gelingt es, eine Orientierung und Position des digitalen Dentalobjekts 100-1 zu ermitteln, die an die Orientierung und Position des Referenzobjekts 100-R im Koordinatensystem angepasst ist.

Das so transformierte digitale Dentalobjekt 100-1 wird im Koordinatensystem des Referenzobjekts 100-R abgespeichert. Das digitale Dentalobjekt 100-1 wird dann in dieser neuen Orientierung und Position im Koordinatensystem abgespeichert und entsprechend für die nachfolgenden Bearbeitungsschritte in der CAM-Software zur Fertigungsvorbereitung bereitgestellt. Nach der fertigungsspezifischen Datenvorbereitung wird dann das reale Dentalobjekt 100-2 hergestellt.

Das Herstellungssystem 200 ist beispielsweise ein 3D-Drucker. Im Allgemeinen ist das Herstellungssystem 200 jedes System, das zum Herstellen des Dentalobjektes geeignet ist. Das Herstellungssystem 200 umfasst beispielsweise einen Computer mit einem digitalen Speicher und einem Prozessor zum Ausführen eines Computerprogramms, durch das einzelne Schritte des Herstellungsverfahrens implementiert werden.

Das Koordinatensystem des Referenzobjekts 100-R ist idealerweise identisch zu dem Koordinatensystem für das Herstellungssystem 200, wie beispielsweise zu dem Koordinatensystem eines Bauraums eines 3D-Druckers. Bei dem 3D-DLP-Druck, einem Stereolithographieverfahren als Herstellungsverfahren, wird das Dentalobjekt 100-2 schichtweise von Unten nach Oben aufgebaut (Bottom Up).

Dabei spannt das gemeinsame Koordinatensystem mit den X- und Y-Achsen die Bauplattform auf und die Z-Achse zeigt von der Bauplattform weg. In diesem Koordinatensystem wird das ausgerichtete digitale Dentalobjekt 100-1 in der CAM-Software zur weiteren Baujobvorbereitung bereitgestellt. Das Bereitstellen schließt unter anderem eine Datenreparatur, die Erzeugung von Stütz- oder Haltestrukturen 103 oder 105, ein Nesting und/oder ein Slicing ein.

Insgesamt erfolgt die automatische und fertigungsspezifische Orientierung des digitalen Dentalobjekts 100-1 dadurch, dass das digitale Dentalobjekt 100-1 mit dem Referenzobjekts 100-R einer passenden Indikation überlagert, angepasst, verglichen und transformiert wird. Die Auswahl oder Bestimmung der dentalen Indikation können auch manuell durchgeführt werden.

Die optimierte Ausrichtung des digitalen Dentalobjekts 100-1 anhand des Referenzobjektes 100-R ermöglicht, dass das Dentalobjekt 100-1 möglichst schnell gefertigt wird, die Gebote und Verbote für Halte- oder Stützstrukturen bestmöglich eingehalten werden, es möglichst wenig Halte- oder Stützstrukturen 103 oder 105 erfordert und indikationsrelevante Flächen des Dentalobjekts 100-2 nicht nachgearbeitet werden müssen.

Durch die CAM-Software kann zahntechnisches Wissen zu optimalen digitalen Fertigungsverfahren implementiert werden. Die CAM-Software sieht eine optimale Orientierung der Dentalobjekte 100-1 für das jeweilige Herstellungsverfahren vor, wie beispielsweise einem additiven oder subtraktiven Herstellungsverfahren. Dabei kann eine Fertigungszeit, eine Ergebnisqualität für eine Passung und Flächengüte und ein minimaler Aufwand bei der Nacharbeit für den Anwender berücksichtigt werden.

Diese Ergebnisse können wiederum in die Datenbank 107, wie beispielsweise in eine Look-Up-Tabelle, zurückgeführt werden, so dass von Fachleuten ein neues dreidimensionales Referenzobjekt 100-R in einer optimalen Position und Orientierung für das Herstellungsverfahren in dem Koordinatensystem abgespeichert wird.

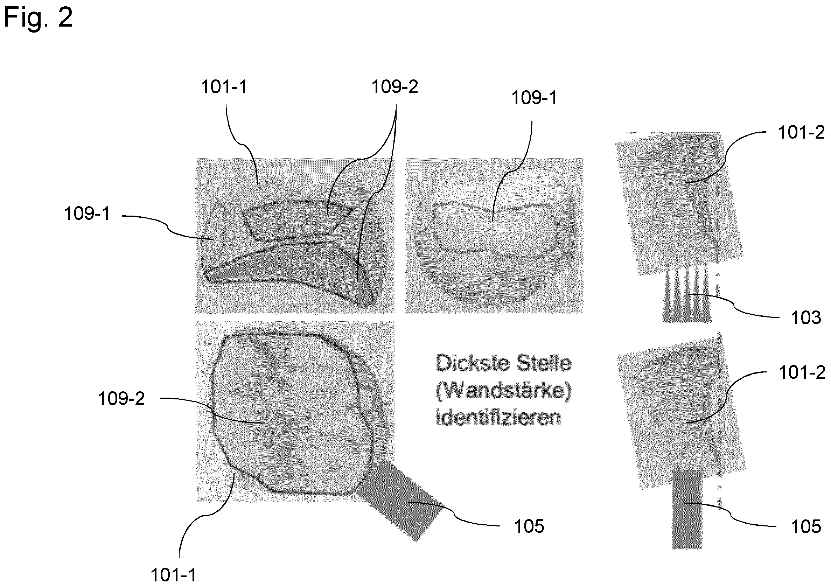

Fig. 2 zeigt ein Dentalobjekt 101-1 mit Stütz- oder Haltestrukturen 103 und 105. Der zahntechnische Arbeitsablauf lässt sich durch die CAM-Software weiter verbessern, indem auch Regeln für die Erzeugung von Stütz- oder Haltestrukturen 103 oder 105 für das Dentalobjekt 101-1 festgelegt werden. Diese Stütz- oder Haltestrukturen 103 oder 105 können von einer Indikation abhängig sein, d.h. indikationsspezifisch sein. Ist die Indikation des importierten Dentalobjektes 101-1 der CAD- oder CAM-Software bekannt, können mit einem entsprechenden Datensatz im weiteren Arbeitsablauf optimierende Maßnahmen durchgeführt werden. Das Referenzobjekt 100-R einer spezifischen Indikation ist bereits so orientiert und positioniert, dass es den zahntechnischen Arbeitsprozess berücksichtig oder optimal unterstützt und die Gebote und -Verbote für Halte- oder Stützstrukturen 103 oder 105 so gut wie möglich eingehalten werden. So ist beispielsweise eine Krone so orientiert, dass die Okklusionsfläche und die Kavität zur Seite zeigen und die dickste Wandstärke zur Bauplattform hin. Okklusionsfläche und Kavität zählen in diesem Fall zu den verbotenen Flächen und die Außenfläche mit der dicksten Wandstärke zur gebotenen Fläche zur Erzeugung der Stützstruktur(en).

Die Stützstrukturen 103 stützen das gefertigte Dentalobjekt 100-2 beim 3D-Drucken an der Bauplattform ab, um einen erfolgreichen Druck zu gewährleisten. Die Haltestrukturen 105 halten das gefertigte Dentalobjekt 100-2 während und nach einem Fräsvorgang innerhalb eines Rohlings in Position.

Beispielsweise können in dem Referenzobjekt 100-R Flächen 109-1 vorgesehen sein, die mit Stütz- oder Halterstrukturen 103 oder 105 belegt werden sollen (Stütz- oder Haltestrukturen-Gebot) oder Flächen 109-2 vorgesehen sein, die nicht mit Stütz- oder Halterstrukturen 103 oder 105 belegt werden dürfen (Stütz- oder Haltestrukturen-Verbot). Durch das zahntechnische Fachwissen und die Kenntnis über den jeweiligen Fertigungsprozess (subtraktiv oder additiv) wird das Referenzobjekt 100-R entsprechend so orientiert und positioniert, dass es die Gebote und Verbote für Stütz- oder Haltestrukturen 103 oder 105 weitestgehend berücksichtigt. Durch die angepasste, automatische Orientierung des digitalen Dentalobjektes 101-1 nach Vorgabe des Referenzobjekts 100-R werden diese Gebote und Verbote indirekt auch bei dem digitalen Dentalobjekt 101-1 durch die neue Orientierung berücksichtigt.

So ergeben sich bei dem Dentalobjekt 101-1 ähnliche, indikations- und fertigungsspezifische Stütz- oder Haltestrukturen 103 oder 105. Die zu verwendenden Parameter für die Stütz- oder Haltestrukturen 103 und 105 können ebenfalls in dem Referenzobjekt 100-R gespeichert sein, wie beispielsweise eine Ausprägung (Dicke, Kontaktfläche) und/oder Dichte/Anzahl der Stütz- oder Haltestrukturen 103 und 105.

Eine CAM-Software kann dann die erforderlichen Stütz- oder Haltestrukturen 103 oder 105 nach ihren Regeln/Algorithmen auf Basis der vorgegebenen Parameter automatisch erzeugen, so dass der Bauauftrag möglichst schnell und stabil mit möglichst wenig Stütz- oder Haltestrukturen 103 oder 105 gefertigt werden kann.

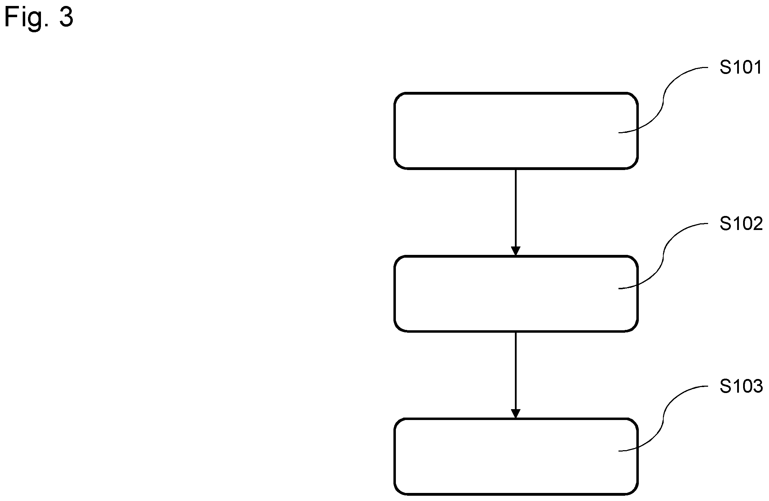

Fig. 3 zeigt ein Blockdiagramm eines Herstellungsverfahrens für ein Dentalobjekt 100-2. Das Herstellungsverfahren umfasst den Schritt eines Erzeugens S101 eines digitalen Dentalobjekts 100-1 in einem Koordinatensystem, das eine Form des herzustellenden Dentalobjekts 100-2 beschreibt. Das digitale Dentalobjekts 100-1 kann beispielsweise in einem CAD-Verfahren (Computer Aided Design) erzeugt werden. In diesem CAD-Verfahren werden die Form und das Aussehen des digitalen Dentalobjekts 100-1 festgelegt. Das digitale Dentalobjekt 100-1 bildet die Grundlage zur Herstellung eines realen Dentalobjekts 100-1.

Bei der 3D-Modellierung wird das digitale Dentalobjekt 100-1 in einer dreidimensionalen Form aufgebaut und gespeichert. Dadurch kann eine realitätsnahe Darstellung erreicht werden. Das digitale Dentalobjekt 100-1 kann als Datensatz gespeichert und übertragen werden, beispielsweise als eine Datei im STL-Format.

Im Schritt S102 wird die räumliche Orientierung des digitalen Dentalobjektes 100-1 in dem Koordinatensystem auf Basis eines digitalen Referenzobjektes 100-R in dem Koordinatensystem neu orientiert und/oder positioniert. Hierzu wird beispielsweise das digitale Referenzobjekt 100-R, das eine definierte Orientierung im Koordinatensystem aufweist, zusammen mit dem digitalen Dentalobjekt 100-1 in einen Speicher geladen. Das digitale Dentalobjekt 101-1 und das Referenzobjekt 100-R werden zuerst durch gleichförmige Skalierung auf eine ähnliche Größe gebracht. Dann wird beispielsweise die Orientierung des digitalen Dentalobjektes 100-R in dem Koordinatensystem gedreht oder die Position des digitalen Dentalobjektes 100-1 in dem Koordinatensystem verschoben, bis eine räumliche Abweichung zwischen dem digitalen Dentalobjekt 100-1 und dem digitalen Referenzobjekt 100-R minimal wird oder einen vorgegebenen Wert unterschreitet.

Im Schritt S103 wird das reale Dentalobjekt 100-2 auf Basis des digitalen Dentalobjekts 100-1 mit der gedrehten räumlichen Orientierung in einem geeigneten Herstellungsverfahren hergestellt.

Das digitale Dentalobjekt 101-1 kann beispielsweise in Abhängigkeit der Indikation und eines ausgewählten Herstellungsverfahrens in einem Rohling, wie beispielsweise beim Fräsen, oder in einem Bauraum, wie beispielsweise beim 3D-Drucken, orientiert werden. Die Orientierung erfolgt so, dass durch die automatisch in der CAM-Software erzeugten Halte- oder Stützstrukturen 103 oder 105 kritische Flächen für die Form und Funktion des realen Dentalobjektes nicht verfälscht werden, ein stabiler Fertigungsprozess garantiert wird und eine Nacharbeit für den Zahntechniker oder Zahnarzt minimiert wird.

Die Zuordnung einer Indikation zu dem digitalen Dentalobjekt 101-1 kann bereits automatisch durch die CAD-Software erfolgen und zum Beispiel in dem Dateinamen enthalten sein, oder durch den Anwender in der CAM-Software nachträglich identifiziert und dem digitalen Dentalobjekt 101-1 zugeordnet worden sein.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100-1: digitales Dentalobjekt
- 100-2: reales Dentalobjekt
- 100-R: digitales Referenzobjekt
- 103: Stützstruktur
- 105: Haltestruktur
- 107: Datenbank
- 109-1: Gebots-Fläche für Stütz- oder Haltestruktur
- 109-2: Verbots-Fläche für Stütz- oder Haltestruktur

- 200: Herstellungssystem

## Patentansprüche

1. Herstellungsverfahren für ein Dentalobjekt (100-2), mit den Schritten:
- Erzeugen (S101) eines digitalen Dentalobjekts (100-1) in einem Koordinatensystem, das eine Form des herzustellenden Dentalobjekts (100-2) beschreibt;
- Drehen (S102) der räumlichen Orientierung des digitalen Dentalobjektes (100-1) in dem Koordinatensystem auf Basis eines digitalen Referenzobjektes (100-R) in dem Koordinatensystem, wobei die Orientierung des digitalen Dentalobjektes (100-R) in dem Koordinatensystem gedreht wird, bis eine räumliche Abweichung zwischen dem digitalen Dentalobjekt (100-1) und dem digitalen Referenzobjekt (100-R) einen vorgegebenen Wert unterschreitet; und
- Herstellen (S103) des Dentalobjekts (100-2) auf Basis des digitalen Dentalobjekts (100-1) mit der gedrehten räumlichen Orientierung.

2. Herstellungsverfahren nach Anspruch 1, wobei eine Größe des digitalen Referenzobjekts (100-R) an eine Größe des digitalen Dentalobjektes (100-1) und/oder eine Größe des digitalen Dentalobjektes (100-1) an eine Größe des digitalen Referenzobjekts (100-R) angepasst wird.

3. Herstellungsverfahren nach einem der vorangehenden Ansprüche, wobei eine Position des digitalen Dentalobjektes (100-1) in dem Koordinatensystem auf Basis eines digitalen Referenzobjektes (100-R) in dem Koordinatensystem verschoben wird.

4. Herstellungsverfahren nach einem der vorangehenden Ansprüche, wobei die Position des digitalen Dentalobjektes (100-1) in dem Koordinatensystem verschoben wird, bis eine räumliche Abweichung zwischen dem digitalen Dentalobjekt (100-1) und dem digitalen Referenzobjekt (100-R) einen vorgegebenen Wert unterschreitet.

5. Herstellungsverfahren nach einem der vorangehenden Ansprüche, wobei das Dentalobjekt (100-2) mittels eines additiven Herstellungsverfahrens hergestellt wird.

6. Herstellungsverfahren nach Anspruch 5, wobei zu dem digitalen Dentalobjekt (100-1) mit der gedrehten räumlichen Orientierung eine digitale Stützstruktur (103) auf Basis der Orientierung des Referenzobjektes (100-R) hinzugefügt wird.

7. Herstellungsverfahren nach Anspruch 6, wobei in dem Referenzobjekt (100-R) spezifiziert ist, welche Flächen mit einer Stützstruktur (103) belegbar sind.

8. Herstellungsverfahren nach einem der vorangehenden Ansprüche, wobei das Dentalobjekt (100-2) mittels eines subtraktiven Herstellungsverfahrens hergestellt wird.

9. Herstellungsverfahren nach Anspruch 8, wobei zu dem digitalen Dentalobjekt (100-1) mit der gedrehten räumlichen Orientierung eine digitale Haltestruktur (105) auf Basis der Orientierung des Referenzobjektes (100-R) hinzugefügt wird.

10. Herstellungsverfahren nach einem der vorangehenden Ansprüche, wobei das digitale Referenzobjekt (100-R) aus einer Datenbank (107) mit mehreren digitale Referenzobjekten (100-R) abrufbar ist.

11. Herstellungsverfahren nach Anspruch 9 oder 10, wobei in dem Referenzobjekt (100-R) spezifiziert ist, welche Flächen mit einer Haltestruktur (105) belegbar sind.

12. Herstellungsverfahren nach einem der vorangehenden Ansprüche, wobei das digitale Dentalobjekt (100-1) Daten über eine Indikation umfasst.

13. Herstellungsverfahren nach Anspruch 12, wobei das digitale Referenzobjekt (100-1) aus einer Datenbank (107) auf Basis der Daten über die Indikation abrufbar ist oder zugeordnet werden kann.

14. Herstellungssystem (200) zum Herstellen eines Dentalobjekts, mit einem Computerprogramm, mit Befehlen, die bei der Ausführung des Computerprogramms durch einen Computer diesen veranlassen, das Verfahren nach einem der Ansprüche 1 bis 14 auszuführen.

## Claims

1. A manufacturing method for a dental object (100-2), comprising the steps of:
- generating (S101) a digital dental object (100-1) in a coordinate system that describes a shape of the dental object (100-2) to be manufactured;
- rotating (S102) the spatial orientation of the digital dental object (100-1) in the coordinate system based on a digital reference object (100-R) in the coordinate system, wherein the orientation of the digital dental object (100-R) in the coordinate system is rotated until a spatial deviation between the digital dental object (100-1) and the digital reference object (100- R) is below a predetermined value; and
- manufacturing (S103) the dental object (100-2) based on the digital dental object (100-1) with the rotated spatial orientation.

2. The manufacturing method according to claim 1, wherein a size of the digital reference object (100-R) is adapted to a size of the digital dental object (100-1) and/or a size of the digital dental object (100-1) is adapted to a size of the digital reference object (100-R).

3. The manufacturing method according to any one of the preceding claims, wherein a position of the digital dental object (100-1) in the coordinate system is shifted based on a digital reference object (100-R) in the coordinate system.

4. The manufacturing method according to any one of the preceding claims, wherein the position of the digital dental object (100-1) in the coordinate system is shifted until a spatial deviation between the digital dental object (100-1) and the digital reference object (100-R) is below a predetermined value.

5. The manufacturing method according to any one of the preceding claims, wherein the dental object (100-2) is manufactured by means of an additive manufacturing method.

6. The manufacturing method according to claim 5, wherein a digital support structure (103) is added to the digital dental object (100-1) with the rotated spatial orientation based on the orientation of the reference object (100-R).

7. The manufacturing method according to claim 6, wherein the reference object (100-R) specifies which areas can be provided with a support structure (103).

8. The manufacturing method according to any one of the preceding claims, wherein the dental object (100-2) is manufactured by means of a subtractive manufacturing method.

9. The manufacturing method according to claim 8, wherein a digital holding structure (105) is added to the digital dental object (100-1) with the rotated spatial orientation based on the orientation of the reference object (100-R).

10. The manufacturing method according to any one of the preceding claims, wherein the digital reference object (100-R) is retrievable from a database (107) comprising a plurality of digital reference objects (100-R).

11. The manufacturing method according to claim 9 or 10, wherein the reference object (100-R) specifies which areas can be provided with a holding structure (105).

12. The manufacturing method according to any one of the preceding claims, wherein the digital dental object (100-1) comprises data about an indication.

13. The manufacturing method according to claim 12, wherein the digital reference object (100-1) is retrievable or assignable from a database (107) based on the data about the indication.

14. A manufacturing system (200) for manufacturing a dental object, comprising a computer program, comprising instructions which, when the computer program is executed by a computer, cause it to execute the method according to any one of claims 1 to 13.

## Revendications

1. Procédé de fabrication d'un objet dentaire (100-2), comprenant les étapes suivantes :
- Génération (S101) d'un objet dentaire numérique (100-1) dans un système de coordonnées décrivant une forme de l'objet dentaire (100-2) à fabriquer ;
- Faire pivoter (S102) l'orientation spatiale de l'objet dentaire numérique (100-1) dans le système de coordonnées sur la base d'un objet de référence numérique (100-R) dans le système de coordonnées, où l'orientation de l'objet dentaire numérique (100-R) dans le système de coordonnées est tournée jusqu'à ce qu'un écart spatial entre l'objet dentaire numérique (100-1) et l'objet de référence numérique (100-R) soit inférieur à une valeur spécifiée ; et
- Fabrication (S103) de l'objet dentaire (100-2) sur la base de l'objet dentaire numérique (100-1) avec l'orientation spatiale tournée.

2. Procédé de fabrication selon la revendication 1, où une taille de l'objet de référence numérique (100-R) est adaptée à une taille de l'objet dentaire numérique (100-1) et/ou une taille de l'objet dentaire numérique (100-1) est adaptée à une taille de l'objet de référence numérique (100-R).

3. Procédé de fabrication selon l'une des revendications précédentes, où une position de l'objet dentaire numérique (100-1) dans le système de coordonnées est décalée sur la base d'un objet de référence numérique (100-R) dans le système de coordonnées.

4. Procédé de fabrication selon l'une des revendications précédentes, où la position de l'objet dentaire numérique (100-1) dans le système de coordonnées est décalée jusqu'à ce qu'un écart spatial entre l'objet dentaire numérique (100-1) et l'objet de référence numérique (100-R) soit inférieur à une valeur spécifiée.

5. Procédé de fabrication selon l'une des revendications précédentes, où l'objet dentaire (100-2) est fabriqué au moyen d'un procédé de fabrication additive.

6. Procédé de fabrication selon la revendication 5, où une structure de support numérique (103) est ajoutée à l'objet dentaire numérique (100-1) avec l'orientation spatiale tournée sur la base de l'orientation de l'objet de référence (100-R).

7. Procédé de fabrication selon la revendication 6, où il est spécifié dans l'objet de référence (100-R) quelles surfaces peuvent être occupées par une structure de support (103).

8. Procédé de fabrication selon l'une des revendications précédentes, où l'objet dentaire (100-2) est fabriqué au moyen d'un procédé de fabrication soustractif.

9. Procédé de fabrication selon la revendication 8, où une structure de maintien numérique (105) est ajoutée à l'objet dentaire numérique (100-1) avec l'orientation spatiale tournée sur la base de l'orientation de l'objet de référence (100-R).

10. Procédé de fabrication selon l'une des revendications précédentes, où l'objet de référence numérique (100-R) peut être récupéré à partir d'une base de données (107) contenant plusieurs objets de référence numériques (100-R).

11. Procédé de fabrication selon la revendication 9 ou 10, où il est dans spécifié l'objet de référence (100-R) quelles surfaces doivent être occupées par une structure de soutènement (105).

12. Procédé de fabrication selon l'une des revendications précédentes, où l'objet dentaire numérique (100-1) comprend des données concernant une indication.

13. Procédé de fabrication selon la revendication 12, où l'objet de référence numérique (100-1) peut être récupéré ou attribué à partir d'une base de données (107) sur la base des données de l'indication.

14. Système de fabrication (200) pour la fabrication d'un objet dentaire, à l'aide d'un programme d'ordinateur, comportant des commandes qui, lorsqu'elles sont exécutées par un ordinateur, amènent le programme d'ordinateur à exécuter le procédé selon l'une des revendications 1 à 13.
